# EUROPÄISCHE PATENTANMELDUNG

(11) **EP 0 867 433 A1**
(43) Veröffentlichungstag der Anmeldung: **30.09.1998**
(21) Anmeldenummer: 98104662.6
(22) Anmeldetag: 16.03.1998
(51) Int. Cl.: C07D 257/04, C07C 253/30, C07C 51/363, C07C 255/50

(54) **Verfahren zur Herstellung von 4-Brommethyl-biphenyl-Verbindungen**

(30) Priorität: 25.03.1997 DE 19712339
(71) Anmelder: Clariant GmbH, 65929 Frankfurt am Main (DE)
(72) Erfinder: Haber, Steffen, Dr., 61462 Königstein (DE)

(57) **Zusammenfassung**

4-Brommethyl-biphenyl-Verbindungen, die in 2'-Stellung durch Cyano, Carboxy, Alkoxycarbonyl von 2 bis 5 C-Atomen, Aminocarbonyl oder gegebenenfalls substituiertes Tetrazolyl substituiert sind, lassen sich mit hoher Reinheit und Ausbeute leicht herstellen, indem man die entsprechende 4-Methyl-biphenyl-Verbindung mit elementarem Brom in Abwesenheit von N-Bromsuccinimid in einem cycloaliphatischen Kohlenwasserstoff umsetzt.

## Beschreibung

4-Brommethyl-biphenyl-Verbindungen der nachstehend angegebenen allgemeinen Formel (1), wie beispielsweise 2-Cyano-4'-(brommethyl)-biphenyl, haben Bedeutung als Zwischenprodukte zur Herstellung von biphenylmethyl-substituierten Imidazolen und Benzimidazolen, die als Pharmawirkstoffe dienen können (J. Med. Chem 39, 625-656 (1996)), aber auch zur Herstellung von Verbindungen, die andere therapeutische Wirkungen besitzen (s. bspw. die japanische Patentanmeldungs-Veröffentlichung Sho-63-23868).

In dieser japanischen Schritt wird die Herstellung von Verbindungen der nachstehend angegebenen und definierten allgemeinen Formel (1) beschrieben, indem man von den korrespondierenden Methyl-biphenyl-Verbindungen ausgeht und diese durch radikalische Bromierung mit N-Brom-succinimid in Anwesenheit eines Radikalkettenstarters, wie Dibenzoylperoxid, zum Brommethyl-Derivat umsetzt. In den europäischen Patentanmeldungs-Veröffentlichungen Nrs. 0 553 879 und 0 595 150 werden desweiteren ähnliche Verfahren unter Verwendung von N-Brom-succinimid beschrieben, gemäß denen die Bromierung in Anwesenheit einer Azobisverbindung zum einen in Methylenchlorid, zum anderen in Chlorbenzol durchgeführt wird. Ein gegenüber diesen europäischen Schritten verbessertes Verfahren ist aus der europäischen Patentanmeldungs-Veröffentlichung Nr. 0 709 369 bekannt, bei welchem die Verwendung des kostenintensiven N-Brom-succinimids durch das kostengünstigere elementare Brom als Bromierungsagenz ersetzt wird und man die Umsetzung außer in chlorsubstituierten aliphatischen und aromatischen Lösemitteln auch in einem Alkan, wie n-Heptan (s. Beispiel 5), durchführt. Gemeinsam aller dieser erstgenannten Verfahren ist die nicht optimale Selektivität der Bromierung von maximal 90 % an dem Monobrom-methyl-Produkt gegenüber dem Dibrom-methyl-Produkt, was auch insbesondere die Reindarstellung der monobromierten, d.h. monobrommethyl-substituierten, Biphenylverbindungen erschwert. Gemäß der Verfahrensweise des Beispieles 5 der europäischen Patentanmeldungs-Veröffentlichung Nr. 0 709 369 erhält man in n-Heptan lediglich eine Ausbeute von 86,4 % an dem Monobrommethyl-Endprodukt, bezogen auf das eingesetzte Ausgangsprodukt.

Mit der vorliegenden Erfindung wurde nun ein Verfahren zur Herstellung von 4-Brommethyl-biphenyl-Verbindungen der allgemeinen Formel (1) 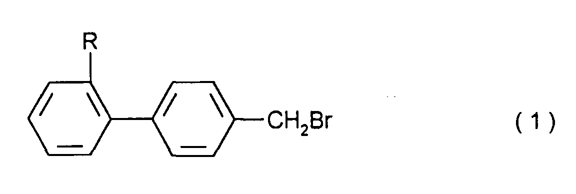 gefunden, in welcher R gleich Cyano, Carboxy, Alkoxycarbonyl von 2 bis 5 C-Atomen, wie Methoxycarbonyl und Ethoxycarbonyl, Aminocarbonyl oder gegebenenfalls substituiertes Tetrazolyl ist, das dadurch gekennzeichnet ist, daß man eine Verbindung der allgemeinen Formel (2) 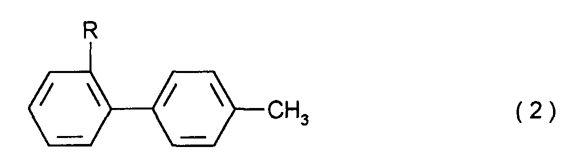 in welcher R die obengenannte Bedeutung besitzt, mit elementarem Brom in Abwesenheit von N-Bromsuccinimid in einem cycloaliphatischen Kohlenwasserstoff, wie beispielsweise einem Cycloalkan von 5 bis 8 C-Atomen, das durch 1 oder 2 Alkylgruppen von 1 bis 4 C-Atomen, wie Methyl und Ethyl, substituiert sein kann, umsetzt.

Mit dem erfindungsgemäßen Verfahren erhält man die Endprodukte der allgemeinen Formel (1) in einer Ausbeute von über 90 %, bezogen auf das eingesetzte Ausgangsprodukt.

Cycloaliphatische Kohlenwasserstoffe als Lösemittel in dem erfindungsgemäßen Verfahren sind beispielsweise Cyclohexan, Methylcyclohexan, Ethylcyclohexan oder Dimethylcyclohexan, wie 1,4-Dimethylcyclohexan, 1,3-Dimethylcyclohexan, 1,2-Dimethylcyclohexan, iso-Propyl-cyclohexan und n-Butyl-cyclohexan, oder Gemische derselben, bevorzugt Methyl-cyclohexan.

Bei dem erfindungsgemäßen Verfahren wird vorzugsweise zur Beschleunigung der Reaktion ein Radikalbildner zugesetzt, wie beispielsweise Verbindungen aus der Gruppe der Azodialkane oder Peroxyde, wie beispielsweise 2,2'-Azo-bis-(4''-methoxy-2'',4''-dimethyl-valeronitril), 2,2'-Azo-bis-isobutyronitril und Dibenzoylperoxid. Die Radikalbildner werden im allgemeinen in einer Menge von 0,1 bis 10 Mol-%, bevorzugt von 0,5 bis 2 Mol-%, bezogen auf die Ausgangsverbindung der allgemeinen Formel (2), eingesetzt.

Das erfindungsgemäße Verfahren kann bei einer Temperatur zwischen 0 und 120°C durchgeführt werden, bevorzugt erfolgt die Umsetzung bei einer Temperatur zwischen 20 und 80°C, insbesondere zwischen 50 und 75°C, da bei niedriger Temperatur die Reaktionsrate zu langsam ist und man bei höheren Temperaturen zunehmend die Bildung von Nebenprodukten beobachtet.

Die Zugabe des elementaren Broms zu der im cycloaliphatischen Kohlenwasserstoff gelösten Verbindung der allgemeinen Formel (2) erfolgt in der Weise in wohldosierten Mengen, daß kein Brom durch den bei der Reaktion freiwerdenden Bromwasserstoff mitgerissen wird.

Das cycloaliphatische Lösemittel wird in der Regel in Verhältnis von 1 bis 20 Gew.-%, bevorzugt von 3 bis 12 Gew.-%, insbesondere bevorzugt von 5 bis 10 Gew.-%, bezogen auf die Ausgangsverbindung der allgemeinen Formel (2), in den Reaktionsansatz eingesetzt.

Nach beendeter Zugabe des elementaren Broms wird der Ansatz noch etwa eine Stunde weitergerührt; die Umsetzung ist sodann beendet. Die hergestellte Verbindung der allgemeinen Formel (1), die aus dem Reaktionsansatz ausfällt, da sie in dem cycloaliphatischen Kohlenwasserstoff nicht löslich ist, und auf diese Weise einer weiteren Bromierung entzogen wird, wird nach beendeter Umsetzung abfiltriert und isoliert. Sie kann ohne weitere Reinigung für nachfolgende Umsetzungen, wie anfangs beschrieben, eingesetzt werden. Die Mutterlauge kann in einen erneuten Prozeß des erfindungsgemäßen Verfahrens zurückgeführt werden, wobei Ausbeuten an dem Endprodukt der allgemeinen Formel (1) von 95 bis 97 %, bezogen auf das Ausgangsprodukt der allgemeinen Formel (2), erzielt werden.

Die Ausgangsverbindungen der allgemeinen Formel (2) sind entweder kommerziell erhältlich oder können anhand bekannter, in der Literatur beschriebener Verfahrensweisen hergestellt werden (s. bspw. J. Med. Chem 34, 2525-2547 (1991)).

Die nachstehenden Beispiele dienen zur Erläuterung der Erfindung.

### Beispiel 1

24,13 g (0,125 Mol) 2-Cyano-4'-methyl-biphenyl werden in 240 g Cyclohexan gelöst; 1,2 g Azo-bis-(isobutyronitril) werden hinzugegeben. Der Ansatz wird auf 60°C erwärmt, und innerhalb von 60 Minuten werden 20 g (0,125 Mol) elementares Brom langsam und stetig hinzugegeben. Anschließend rührt man noch etwa eine Stunde bei 60°C nach und saugt das ausgefallene Produkt ab.
Man erhält 30,6 g (0,113 Mol) entsprechend 90,4 % d.Th. 2-Cyano-4'-(brommethyl)-biphenyl mit einer Reinheit von 98 % gemäß gaschromatographischer Analyse.

### Beispiel 2

48,2 g (0,25 Mol) 2-Cyano-4'-methyl-biphenyl werden in 500 g Methylcyclohexan gelöst. 0,48 g 2,2'-Azo-bis-(2''-methyl-propionitril) werden hinzugegeben und nach Erwärmung auf 60°C werden zu dem Reaktionsansatz innerhalb von zwei Stunden langsam und stetig 48 g Brom hinzugegeben. Man rührt den Ansatz noch eine Stunde nach und isoliert das ausgefallene 2-Cyano-4'-(brommethyl)-biphenyl durch Filtration in einer Ausbeute von 63,2 g (gleich 0,23 Mol entsprechend 92 % d.Th.) mit einer Reinheit von 98,3 % gemäß gaschromatographischer Analyse.

## Patentansprüche

1. Verfahren zur Herstellung einer Verbindung der allgemeinen Formel (1) 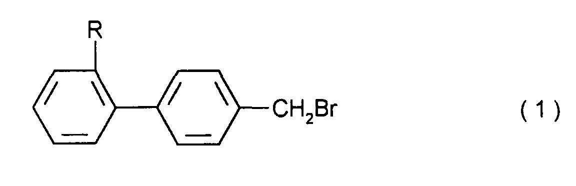 in welcher R gleich Cyano, Carboxy, Alkoxycarbonyl von 2 bis 5 C-Atomen, Aminocarbonyl oder gegebenenfalls substituiertes Tetrazolyl ist, dadurch gekennzeichnet, daß man eine Verbindung der allgemeinen Formel (2) 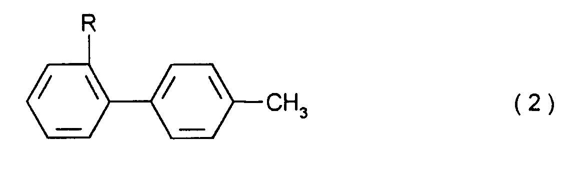 in welcher R die obengenannte Bedeutung hat, mit elementarem Brom in Abwesenheit von N-Brom-succinimid in einem cycloaliphatischen Kohlenwasserstoff umsetzt.

2. Verfahren nach Anspruch 1, dadurch gekennzeichnet, daß man die Umsetzung in Gegenwart eines Radikalbildners durchführt.

3. Verfahren nach Anspruch 1 oder 2, dadurch gekennzeichnet, daß der cycloaliphatische Kohlenwasserstoff Cyclohexan, Methylcyclohexan, Ethylcyclohexan oder ein Dimethylcyclohexan ist.

4. Verfahren nach Anspruch 1 oder 2, dadurch gekennzeichnet, daß der cycloaliphatische Kohlenwasserstoff Methylcyclohexan ist.

5. Verfahren nach mindestens einem der Ansprüche 1 bis 4, dadurch gekennzeichnet, daß man die Umsetzung bei einer Temperatur zwischen 20 und 80°C, bevorzugt zwischen 50 und 75°C, durchführt.
